# EUROPEAN PATENT SPECIFICATION

(11) **EP 4 366 804 B1**
(45) Date of publication and mention of the grant of the patent: **29.04.2026**
(21) Application number: 22741592.4
(22) Date of filing: 01.07.2022
(51) Int. Cl.: A61M 15/00, A61K 9/00

(54) **A DRY-POWDER NASAL INHALER COMPRISING A CASING WITH A FIRST CASING PORTION AND A SECOND CASING PORTION**
NASALER TROCKENPULVERINHALATOR MIT EINEM GEHÄUSE MIT EINEM ERSTEN GEHÄUSETEIL UND EINEM ZWEITEN GEHÄUSETEIL
INHALATEUR NASAL DE POUDRE SÈCHE COMPRENANT UN BOÎTIER AVEC UNE PREMIÈRE PARTIE DE BOÎTIER ET UNE SECONDE PARTIE DE BOÎTIER

(30) Priority: 06.07.2021 SE 2150886
(43) Date of publication of application: 15.05.2024
(73) Proprietor: Iconovo AB, 223 70 Lund (SE)
(72) Inventor: LASTOW, Orest, 247 45 TORNA HÄLLESTAD (SE); KARLSSON, Simon, 227 30 LUND (SE)
(74) Representative: Ström & Gulliksson AB
(86) International application number: PCT/SE2022/050671
(87) International publication number: WO 2023/282821

(56) References cited:
- EP-A1- 2 015 812
- EP-B1- 1 351 735
- DE-A1- 102014 005 646
- US-A1- 2008 190 424

## Description

### Field of the Invention

This disclosure pertains in general to the field of medicament inhalers, and according to the invention to dry powder inhalers. The inhaler comprises a casing comprising a first casing portion, a second casing portion and a hinge arrangement connecting said first casing portion and second casing portion, whereby the inhaler is assembled by means of folding the first casing portion and the second casing portion together. The inhaler further comprises at inlet and an outlet extending at an angle from the casing.

### Background of the Invention

In the pharmaceutical field, with respect to vaccine administration or treatment of respiratory and/or other diseases, inhalers have been widely used. Numerous drugs, vaccines, medications and other substances are inhaled into the lungs or into the nasal tract through nasal administration for rapid absorption in the blood stream and for local action at the site of administration.

Inhaled drugs fall into two main categories, in form of liquids, including suspensions, and powders. The choice of category depends on the characteristics of the drugs, medications, etc., to be inhaled.

The most common type of inhaler is the pressurized metered-dose inhaler. In this type of inhaler medication is most commonly stored in solution in a pressurized canister that contains a propellant, although it may also be a suspension. The canister is attached to a plastic, hand-operated actuator. On activation, the metered-dose inhaler releases a fixed dose of medication in aerosol form.

Another kind of inhaler is a nebulizer, which supply medication as an aerosol created from an aqueous formulation.

The kind referred to herein is yet another type, in form of a dry powder inhaler. A dry powder inhaler releases a pre-metered, capsuled dose or a device-metered dose of powdered medication that is inhaled through the inhaler. Inhalers with device-metered dose of powdered medication is normally inhalers with medication reservoir, containing powdered medication, from which metered doses are withdrawn through the use of different dose metering arrangements, said doses then being inhaled.

Unit dose dry powder inhalers are commonly used in order to ensure the hygiene as well as the proper dose of medicament being provided to the user. Unit dose dry powder inhalers contain an encapsulated dose and are disposed of after the dose has been inhaled.

Further, said inhalers does not require any dose-meter arrangements since they only contain one dose which makes them considerably less expensive to manufacture due to their less complex design.

Due to their low cost and one-dosage functionality, the unit dose dry powder are usually manufactured in large volumes, often by means of injection molding.

To guarantee the function of the inhaler, said inhaler requires the provision of several components inside its casing, such as blister foils, flow directing elements for a more optimal de-aggregation etc. Furthermore, the reservoir for containing the dose of medicament has to be filled with the appropriate dose.

Hence, the casing of the unit dose dry powder inhalers are traditionally manufactured in at least two separate casing parts, whereby the casing parts are provided with the necessary components and dosage.

Due to the large production volumes usually associated with the production of such one dose dry powder inhalers the consequent joining and handling of the multiple casing parts makes the manufacturing and assembly considerably more complex as well as more expensive.

A known drawback with dry powder inhalers is that the device must be kept at a certain orientation, e.g. a horizontal orientation, during administration to prevent the powdered drug from becoming displaced inside the inhaler. This is especially an issue when nasal administration is a preferred administration route, since it is difficult for the user to place the inhalation nozzle in the nasal vestibule without turning or disorienting the inhaler.

In view of these drawbacks and limitations of the prior art, what is needed is a dry powder inhaler for nasal administration of a powdered drug or vaccine, which can be assembled and manufactured in a less complex and more cost-efficient manner.

The following documents are disclosing different dry powder inhalers, namely US 2008/190424 A1, EP 2 015 812 A1 and DE 10 2014 005646 A1.

### Summary of the Invention

The invention is disclosed in the appended claims. Accordingly, the present disclosure preferably seeks to mitigate, alleviate or eliminate one or more of the above-identified deficiencies in the art and disadvantages singly or in any combination and solves at least the above mentioned problems by providing a dry-powder inhaler comprising a casing, the casing comprising a first casing portion, a second casing portion and a hinge arrangement connecting said first casing portion and second casing portion; whereby the dry-powder inhaler is assembled by means of folding the first casing portion and the second casing portion together; whereby the first casing portion comprises a cavity for receiving the dose blister foil, said cavity being adapted to contain a dry-powder medicament; and whereby the first casing portion and second casing portion when folded together are adapted to form an inlet for allowing a lidding foil extending out there from and whereby the casing further comprises an outlet nozzle for providing the medicament, said outlet nozzle extending at an angle from one of said first and second casing portions.

Additionally, the present disclosure preferably seeks to mitigate, alleviate or eliminate one or more of the above-identified deficiencies in the art and disadvantages singly or in any combination and solves at least the above mentioned problems by providing a method for providing a dry-powder inhaler, whereby the method comprises providing a casing, the casing comprising a first casing portion, a second casing portion and a hinge arrangement connecting said first casing portion and second casing portion, the first casing portion and second casing portion being adapted to, when folded together, form an inlet for allowing a lidding foil extending out there from and an outlet for providing the medicament; and assembling the inhaler by means of folding the first casing portion and second casing portion together.

Further advantageous embodiments are disclosed in the appended and dependent patent claims.

### Brief Description of the Drawings

These and other aspects, features and advantages of which the disclosure is capable will be apparent and elucidated from the following description of embodiments of the present invention, reference being made to the accompanying drawings, in which
Fig. 1 is a perspective view of the inhaler of one embodiment without a lidding foil and the casing unfolded;
Fig. 2 is a perspective view of the inhaler of one embodiment without a lidding foil and the casing unfolded;
Fig. 3 is a perspective view of the inhaler of one embodiment with a lidding foil and the casing unfolded;
Fig. 4 is a longitudinal cross-section of the inhaler of one embodiment without a lidding foil and the casing folded;
Fig. 5 is a perspective view of the inhaler of one embodiment without a lidding foil and the casing folded; and
Fig. 6 is a perspective view of the inhaler of one embodiment without a lidding foil and the casing folded, seen from below.

### Description of Embodiments

The following description focuses on an embodiment of the present disclosure applicable to a medicament inhaler, and in particular a dry powder inhaler. However, it will be appreciated that the invention is not limited to this application but may applied to many other inhalers having an inlet and an outlet, as well as a medicament reservoir.

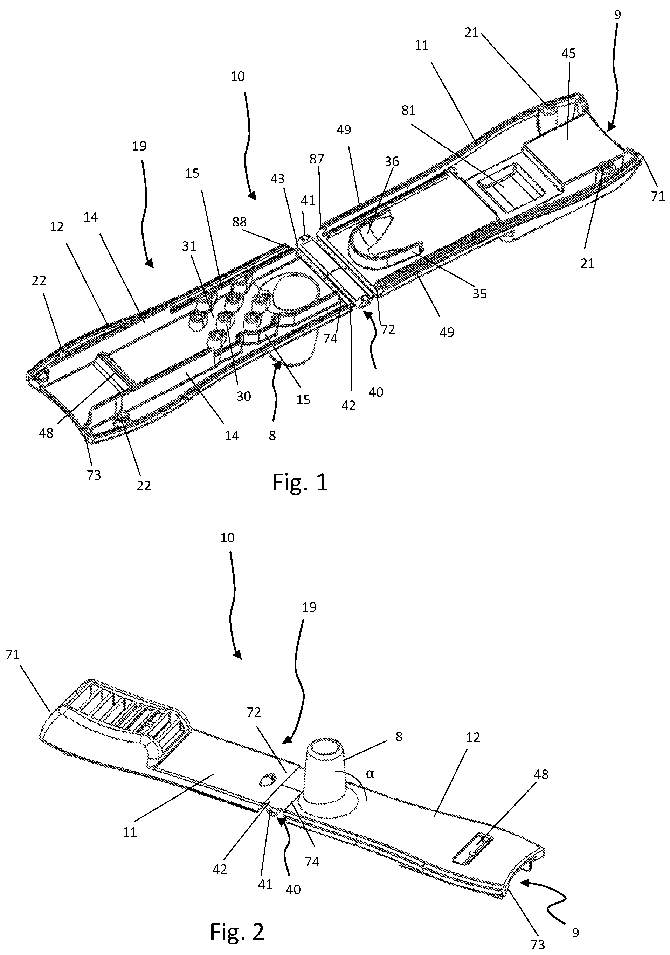

Fig. 1 shows a dry-powder inhaler 10. The dry-powder inhaler 10 comprises a casing 19, the casing preferably forming the outer shape of the dry-powder inhaler 10.

The casing 19 comprises a first casing portion 11 and a second casing portion 12, whereby the casing 19 further comprises a hinge arrangement 40 connecting the first casing portion 11 and the second casing portion 12. The casing 19 further comprises an inlet 9, and an outlet 8, said outlet 8 being in the form of a tubular, cylindrical nozzle. Hence, a transverse cross-section of the tubular outlet 8 is circular. Further, the outlet 8 is preferably tapered, being wider adjacent to the casing 19 and more narrow towards the outer end of the nozzle outlet 8. The outlet 8 extends at an angle α (shown in Fig. 2) from the casing 19. In Fig. 2 the outlet extends in a direction perpendicular to the longitudinal extension of the casing 19. However, the outlet 8 may alternatively extend at an angle α being larger than 45 degrees, preferably between 45 and 90 degrees, such as 50, 60, 70, 80 or 90 degrees.

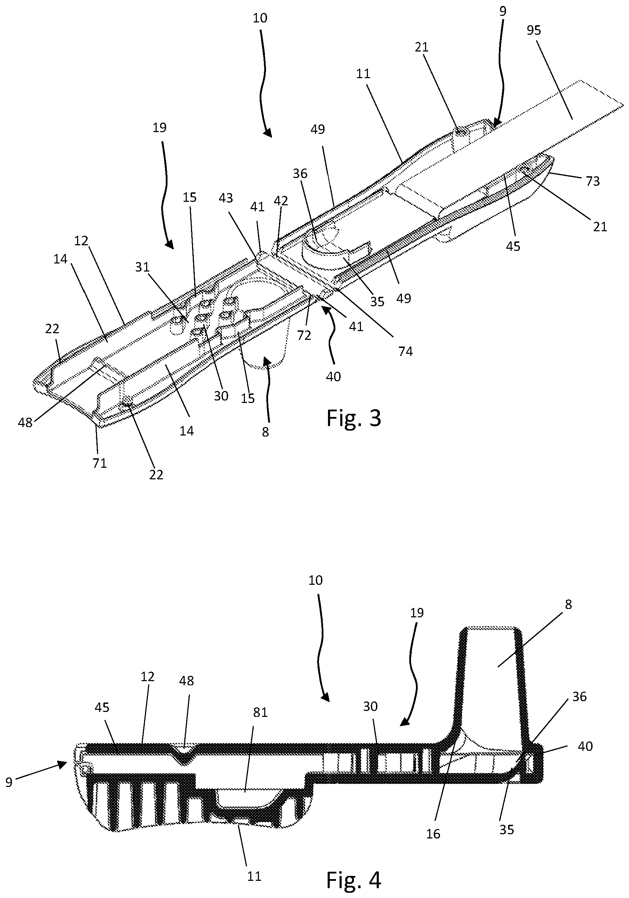

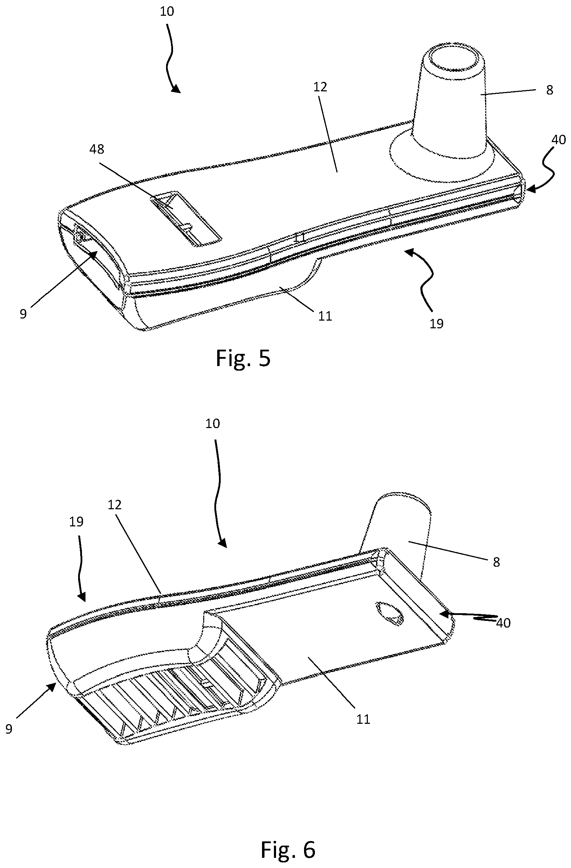

The upper edges of the nozzle outlet 8 are preferably rounded (shown in Figs 4 and 5) to make the use of the inhaler 10 comfortable for the user. However, the nozzle outlet 8 may have a cross-sectional shape other than a circular shape, such as elliptic, rectangular, triangular or any other shape enabling insertion of the nozzle outlet 8 in a nasal vestibule.

Referring to said figure, the first casing portion 11 comprises a cavity 81 for receiving a lidding foil (shown in Fig. 3), the cavity 81 being adapted to contain the dry-powder medicament (such as a drug or a vaccine substance). This may for example be achieved by means of the lidding foil being heat sealed, i.e. welded, to the surrounding walls of the cavity 81 so as to seal away the cavity 81 from the rest of the inside of the casing 19 and protect the medicament from moisture. Thereby, the dry-powder medicament may be kept in place during transport and handling of the inhaler 10 without any risk for leakage of dry-powder medicament through the inlet 9 and the outlet 8 of the casing 19.

By means of the hinge arrangement 40 the inhaler 10 may be assembled by folding the first casing portion 11 and the second casing portion 12. The first casing 11 and the second casing portion 12 may thus when folded together be adapted to form an inlet 9 for allowing the lidding foil 95 extending out there from. The outlet 8 is configured for providing the medicament to the nasal tract of the user through nasal administration.

As is conventional the user may thus operate the inhaler 10 by means of first removing the lidding foil 95 so as to expose the dry-powder medicament therein. The user may then inhale the medicament by nasal administration by placing the nozzle outlet 8 in the nasal vestibule and inhaling thus allowing for the dry-powder medicament to flow through the casing 19 of the inhaler 10 towards the user, whereby the inlet 9 allows air to enter the casing 19 and force the dry-powder medicament towards the outlet 8. The user may thus inhale the dry-powder medicament provided inside cavity 81 of the casing 19 into his/her nasal vestibule and nasal tract, whereby the treatment is finished and the inhaler 10 may be disposed of.

Since the outlet nozzle 8 extend transversally from the casing 19, the user does not need to turn the inhaler 10 during nasal administration. Hence, the casing 19 may be kept in a horizontal position while the user inserts the outlet 8 into the nasal vestibule. This prevents displacement of the powdered medicament inside the casing and facilitates a successful nasal inhalation where a maximum amount of the medicament is administered to the user.

The casing 19, which preferably may be in a plastic material such as PP or PE, may be manufactured by means of injection moulding. Due to the casing 19 being in one piece during at least the majority of the assembling due to the hinge arrangement 40 interconnecting the first casing portion 11 and the second casing portion 12 the transportation and assembly is made considerably less complex.

The hinge functionality 40 allows for the for the casings 19 to be transported and delivered in a simple manner since the casing portions 11, 12 may be connected prior to the insertion of the remaining components such as flow directing elements and lidding foils.

This is particularly advantageous due to the filling of the medicament and the sealing of said medicament inside the cavity 81 by means of the lidding foil 95 usually being performed at another location than the manufacturing of the casing 19. By connecting the casing portions 11, 12 prior to distribution to a site where filling and sealing medicament, the number of components to be handled during assembly and transport is reduced considerably, which allows for a more cost-efficient handling and transportation.

Further, the interconnected casing portions 11, 12 allows for a much more user-friendly and cost-efficient final assembly and filling of medicament since the filling and sealing operation does not require any complex and time consuming joining operations to be performed. Instead, the casing 19 may just be folded together after being "loaded" with the dry-powder medicament.

The outlet nozzle 8 for nasal administration is also injection moulded in one piece together with the casing portions 11, 12 of the casing 19, and the inlet 9 is formed when the first casing portion 11 and the second casing portion 12 are folded together. Thereby, the entire casing may be manufactured in one production step which decreases the complexity and cost for assembling and manufacturing considerably compared to a conventional inhaler where the separate portions have to be handled separately and then mounted together.

The enablement of injection moulding of the entire casing in one piece further makes the casing far less susceptible for any tolerance errors since each cavity the casing is individual. Thereby, the first casing portion 11 only needs to fit with the second casing portion 12 moulded in the same cavity. In a conventional injection moulding process where the casing portions are injection moulded separately each first casing portion needs to fit with each second casing portion, this may lead to several casing portions having to be disposed of due to tolerance errors disallowing proper assembling. Hence, the enablement of injection moulding the entire casing in one piece allows for a more cost-efficient and reliable manufacturing process.

Further referring to Fig. 1, the inlet 9 may be formed by the respective ends of the casing portions 11, 12 being provided with cut-outs, whereby said cut-outs are adapted to together form said inlet 9 when the casing is in the folded position. The hinge arrangement 40 is disposed between said first casing portion 11 and the second casing portion 12 so as to form a pivot axis.

Accordingly, the first casing portion 11 comprises a first end 71 and a second end 72, whereby the second casing portion 12 comprises a corresponding first end 73 and corresponding second end 74. The second ends 73, 74 are connected by means of the hinge arrangement 40.

The inlet 9 may thus be formed by the first end 71 of the first casing portion 11 and the corresponding first end 73 of the second casing portion 12 when the casing is in the folded position, as shown in Fig. 2. Each of the first end 71 and the corresponding first end 73 may comprise a cut-out extending along a plane orthogonal to a longitudinal axis of the inhaler 10, whereby the cut-outs together form the inlet 9 when the casing is in the folded position.

Again referring to Fig. 1, the first casing portion 12 further comprises a protruding shoulder 45 i.e. a shoulder which protrudes from the first casing portion 11 towards the second casing portion 12 when the casing 19 is in a folded position, whereby said protruding shoulder 45 is adapted to receive the lidding foil 95 (shown in Fig. 3).

The protruding shoulder 45 is preferably disposed between the inlet 9 and the cavity 81. Preferably, said protruding shoulder 45 is disposed between the inlet 9 and the cavity 81 so as to together with the second casing portion 12 form the inlet 9 when the casing 19 is in a folded position.

This is particularly advantageous since the protruding shoulder 45 may counteract the inherent hollow shape of the casing 19 around the inlet 9 in the folded position so as to achieve a more smooth transition between the casing portions 11, 12 around said inlet 9. Without the protruding shoulder 45 there is a risk for the air flow becoming turbulent due to the casing 19 forming a steep diverging portion just downstream of the inlet 9. This may cause some of the air flow to just pass by the cavity 81, whereby a larger air flow is required in order to de-aggregate the dry-powder medicament inside the casing 19. The protruding shoulder 45 however reduces the diverging flow effect by directing the flow of air entering the inlet 9 and decreasing the diverging angle around the inlet 9, whereby a more efficient de-aggregation is achieved.

The inhaler 10 may further comprise at least one but preferably a plurality of flow directing elements 30 adapted to disaggregate the medicament. Hence, the inhaler 10 comprises the flow directing elements 30 being disposed inside the casing 19 between the cavity 81 and the outlet 8 of the inhaler 10 when the inhaler 10 is in its folded configuration. In other words, the flow directing elements 30 may be disposed downstream of the cavity 81 and upstream of the outlet 8 when the casing 19 is in a folded position.

The provision of the flow directing elements 30 allows for directing of the air flow straight through the inhaler 10, i.e. from the inlet 9 to the outlet 8 for the medicament which achieves a more laminar flow pattern of air. This allows for more air to pass through the entire inhaler 10 with a single inhale, whereby a lower aggregation of medicament is achieved, hence increasing the potential of the medicament to reach out far in the nasal tract of the patient.

Additionally, the impinging effect on the air flow due to said flow directing elements 30 causes the powder contained in the air flowing through the inhaler 10 during inhalation to collide with said flow directing elements 30 and disperse even further, whereby an even lower aggregation may be achieved.

Said flow directing elements 30 may be integrated elements protruding from the first and/or second casing portion. The flow directing elements 30 may also be provided on a flow directing element insert 31, preferably in the shape of a plate to be received and attached to the first and/or second casing portion.

With advantage, the flow directing elements 30 may protrude from the first casing portion 11, i.e. the casing portion comprising the cavity 81. Due to the foldable design of the inhaler 10 a small gap is required between the flow directing elements and the casing portion which is not provided with said flow directing elements 30 to ensure the ability to fold and interlocking of the two casing portions 11, 12.

To optimise and maximise the disaggregation of the medicament, the flow directing elements 30 are substantially drop shaped with their tapered end extending towards the outlet.

In addition, the first casing portion 11 comprises a flow directing shoulder 35. The shoulder is horse shoe shaped and extends upwardly from an interior surface of the first casing portion 11. When the casing 19 is in the folded state, the flow directing shoulder 35 will be positioned adjacent the periphery of the nozzle outlet 8, such that the medicament is directed upwards and into the tubular nozzle outlet 8. A sloped interior surface 36 of the flow directing shoulder 35 further contributed to the directing effect of the shoulder 35 such that the powder medicament easily flows into the tubular nozzle outlet 8. The flow directing shoulder 35 also prevents the powdered medicament from passing the outlet 8 without being directed into the nozzle outlet 8 and inhaled nasally by the user.

The second casing portion 12 comprises a set of inner walls 14 protruding from said second casing portion 12, the set of inner walls 14 extending parallel along the second casing portion 12, whereby each of the inner walls of the set of inner walls 14 has at least one inwardly directing portion 15 adapted to direct a flow inwardly towards the outlet 8. Thus the air flowing through the inhaler 10 may converge towards a longitudinal center axis of the inhaler, this reduces the risk for turbulence due to impinging effects in the vicinity of the outlet 8, i.e. due to the walls surrounding the outlet 8. More air is thus allowed to pass through the entire inhaler 10, i.e. from the inlet 9 to the outlet 8 during the inhalation, which reduces the aggregation even further, allowing for more of the dry-powder medicament to enter the nasal vestibule/tract of the user.

The inner walls 14 extend one on each side along a longitudinal centerline of the inhaler 10 along the entire length of the casing 19 when the casing 19 is in a folded position. Said inwardly directing portion 15 may be formed by the inner walls 14 having at least one tapered portion with a tapering angle directed diagonally inwardly towards the outlet 8 when the casing is in the folded position. As shown in Fig. 1, the inwardly directing portions 15 each have two tapering angles directed diagonally inwardly towards the outlet 8 when the casing is in the folded position.

As the skilled person realizes said set of inner walls 14 may also be provided on the first casing portion 11, whereby the first casing portion 11 may comprise a set of inner walls 14 according to the above. Said second casing portion 12 may further comprise a flow directing member 48 adapted to direct flow entering through the inlet 9 towards the cavity 81. Thus the air entering the inlet 9 is forced towards the cavity 81 of the first casing portion 11 whereby the cavity 81 may be efficiently emptied of dry-powder medicament due to the dispersion effect of the air impinging on the bottom surface of the cavity 81. Thus an initial de-aggregation of the dry-powder medicament is achieved right away during inhalation which reduces the risk for dry-powder medicament getting stuck in the cavity 81 as well as the risk for insufficient de-aggregation of medicament through the inhaler 10.

As shown in said Fig, 1, the flow directing member 48 may be a fin extending orthogonal to the longitudinal axis of the inhaler 10 i.e. along the width of the second casing portion 12, said fin may accordingly have an angular face adapted to impinge on the air moving through the inhaler 10 so as to direct it towards the cavity.

Preferably, said flow directing member 48 may extend between the inner walls 14 defining the longitudinal delimitations of the flow through area of the inhaler 10.

To retain the first casing portion 11 and the second casing portion 12 together in the folded position one of the casing portions 11, 12 may comprise at least one latching knob 22, whereby the other casing portion 11, 12 may comprise at least one aperture 21. The at least one aperture 21 is adapted to receive the at least one latching knob 22, so as to retain the casing portions 11, 12 in a folded position. Thereby the casing portions 11, 12 may be latched together simply by the retaining properties of the material of the casing 19 by means of the elastic deformation of the knob(s) 22 and/or the material forming the aperture(s) 21, whereby no additional mechanical fastening elements such as screws are required, resulting in a less complex and more cost-efficient assembling process.

Further referring to Fig. 1, the first casing portion 11 may be provided with a pair of apertures 21 for receiving a pair of knobs 22 provided on the second casing portion 12 when the casing is in a folded position. As depicted in said figure, the pair of knobs and the pair of apertures may be disposed on the respective casing portion so as to be on each side of the inlet 9 when the casing 19 is in the folded position.

Referring to Fig. 1 as well as Fig. 2, the hinge arrangement 40 comprises a folding portion 42 comprising support members 41 protruding therefrom, whereby the support members 41 are adapted to abut to the first casing portion 11 and the second casing portion 12 when said casing portions 11, 12 are folded together. The folding portion 42 accordingly defines the pivot axis of the casing 19, whereby the support members 41 are disposed parallel along the pivot axis defined by said folding portion 42. Said support members 41 guarantees a set distance between the first casing portion 11 and the second casing portion 12 when the casing 19 is in a folded position as a result of the abutting contact between the first casing portion 11 and the support members 42 and the second casing portion 12 and the support members 42.

The support members 42 define the set space forming the height of a lumen between the first casing portion 11 and the second casing portion 12 in the folded position. The support members 42 may each have a first phase 43 of the adapted to abut to the first casing portion 11 when the casing 19 is in a folded position and a second phase 43 adapted to abut to the second casing portion 12 when the casing 19 is in a folded position. The first and second phase 43 of each support block 42 being parallel opposite surfaces.

With reference to Fig. 1 and 3 the support elements 30 may have a substantially trapezoidal shape, whereby the bottom phases of the support elements 30 are substantially wider than the top phases, i.e. the protruding phases of the support elements 30. The bottom phases preferably being aligned with the first casing portion 11 and the second casing portion 12 when the casing is in a fully unfolded state. The first casing portion 11 may accordingly comprise diagonal wall phases 87 adapted to abut to the diagonal phases 43 of support elements 41. Correspondingly, the second casing portion 12 may comprise diagonal wall phases 88 adapted to abut to the corresponding diagonal phases 43 of the support elements 41. Thus, a sufficient sealing is achieved in the joints between the casing portions and the support elements.

Referring to Fig. 2, exterior surfaces of the casing 19 of the inhaler 10 is shown in an unfolded position. The first casing portion 11 has the first end 71 and the second end 72, and the second casing portion 12 comprises the corresponding first end 73 and corresponding second end 74. The second ends 72, 74 are connected by means of the hinge arrangement 40 comprising the support member 41 and the folding portions 42. Said folding portions 42 may in turn comprise a pair of folding elements, each of which extending between and thereby interconnecting the first casing portion 11 and the second casing portion 12.

Preferably, the support members 41 may be a pair of support members 41 which are formed by a pair protrusions one of each extending from the folding portion, i.e. from each of the pair of folding elements. Thereby, the hinge arrangement 40 may be injection molded in one piece, whereby the entire casing 19 and the entire hinge arrangement 40 may be molded in one piece. This further reduces the cost of the manufacturing of the inhaler as well as reduces the risk for tolerance errors in the manufacturing process.

The outlet 8 is arranged at the second end 74 of the second casing portion 12 and has a substantially cylindrical shape. The outlet nozzle 8 has a height sufficiently long to fit into the nasal vestibule of the user. The nozzle outlet 8 extends at the angle α relative the plane of the casing 19. In Fig. 2, the nozzle outlet 8 extends substantially perpendicularly, i.e. transversally, in relation to the longitudinal extension of the inhaler 10, from the second casing portion 12. However, as stated earlier, the outlet 8 may extend at an angle α of 50, 60, 70, or 80 degrees relative to the plane of the casing 19. Further, the outlet nozzle 8 is slight tapered, having a broader width adjacent to the second casing portion 12 and being less wide at its outer end. This shape facilitates the fitting of the nozzle 8 into the nasal vestibule of the user and makes the outlet nozzle 8 more comfortable for the user.

Referring to Fig. 3, to seal the medicament inside the medicament reservoir, the dry-powder inhaler 10 may comprise a lidding foil 95 being removably attached to the first casing portion 11 to seal the cavity 81 adapted to contain a dry-powder medicament. The lidding foil 95 may preferably be made of aluminium, due to its advantageous sealing properties.

With advantage, the lidding foil 95 may be removably attached to the first casing portion 11 by means of heat sealing. However, mechanical fastening means such as a clamp arrangement may be applicable as well.

Further, said lidding foil 95 may also be removably attached to the protruding shoulder 45 of the first casing portion 11, preferably by means of glue.

Fig. 4 shows a longitudinal cross-section of the inhaler 10 and its casing 19. The casing 19 is in its folded position with the hinge arrangement 40 connecting the first casing portion 11 and the second casing portion 12. As clearly shown in Fig. 4, the outlet nozzle 8 has a tapered shape and extends transversally from the second casing portion 12. No lidding foil 95 is arranged on top of the should portion 45 in Fig. 4. Thus, the cavity 81 is open and the medicament housed therein can be inhaled by nasal administration through the outlet 8. When the user inhales the medicament, the flow directing elements 30 and the flow directing shoulder 35 guide the medicament towards the outlet 8. As shown in Fig. 4, the sloped internal surface 36 of the flow directing shoulder 35 abuts the edge of the outlet 8 facing the second ends 72, 74 of the casing portions 11, 12. Further, the outlet 8 has a rounded, sloped interior surface 16 which extends into the lumen of the tubular nozzle outlet 8. Together, the rounded interior surface 16 and the sloped interior surface 36 of the flow directing shoulder 35 forms a rounded deflection directing the powdered medicament into the nozzle outlet 8.

With reference to Fig. 5, which depicts the casing 19 of the inhaler 10 in a folded state, the first casing portion 11 may be positioned so as to be a bottom portion during the assembling of the inhaler 10. In Fig. 5, the casing 19 is viewed from below.

This enables simple dosage of the dry-powder medicament which can be dosed into the cavity 81 of the bottom casing portion, i.e. the first casing portion 11. Thereafter the blister foil 95 may be provided, whereby said blister foil 95 may be heat sealed to the cavity, i.e. the walls surrounding the cavity and the protruding shoulder 45.

At this stage the blister foil 95 is positioned so as to extend on top of said cavity 81 and a significant distance outwardly from the first end 71 of the first casing portion 11 partially forming the inlet 9.

Furthermore, the flow directing elements 30 may be attached to the first casing portion 11. Preferably this is performed by means of the flow directing elements 30 being provided on a flow directing element insert 31 adapted to be fastened to the first casing portion 11. Said flow directing element insert 31 being a plate for fastening to the first casing portion 11, whereby the flow directing elements 30 protrude towards the second casing portion 12 from the insert 31 when the casing is in the folded position.

Finally, the casing portions may be folded together, whereby the casing portions are brought into contact with each other thus forming the casing of the inhaler 10.

Referring to Fig. 6, the casing portion 19 in a fully folded state is shown in a perspective view seen from above. As seen in said figure, the outlet 8 extends transversally, substantially perpendicular, from the second casing portion 12.

Further referring to Fig. 6, the flow directing member 48 may be formed by a an inward protrusion in the form of a dimple extending into the inner of the casing 19, i.e. towards the first casing portion 11 when the casing 19 is in the folded position.

The present disclosure may further relate to a method for providing a dry-powder inhaler, whereby the method comprises providing a casing 19, the casing 19 comprising a first casing portion 11, a second casing portion 12 equipped with a nozzle outlet 8 for providing the medicament and a hinge arrangement 40 connecting said first casing portion 11 and second casing portion 12, the first casing portion 11 and second casing portion 12 being adapted to, when folded together, form an inlet 9 for allowing a lidding foil 95 extending out there from; the method further comprising assembling the inhaler 10 by means of folding the first casing portion 11 and second casing portion 12 together.

The method may also comprise connecting the first casing portion 11 and the second casing portion by means of the hinge arrangement 40 so as to form the casing 19, prior to the folding of said casing 19.

Alternatively, the method may comprise injection molding the casing 19 prior to the folding of said casing 19, whereby the first casing portion 11, the second casing portion 12 and the hinge arrangement 40 are integrated parts of said casing portion 18.

Although, the present invention has been described above with reference to specific embodiments, it is not intended to be limited to the specific form set forth herein. Rather, the invention is limited only by the accompanying claims.

In the claims, the term "comprises/comprising" does not exclude the presence of other elements or steps. Furthermore, although individually listed, a plurality of means, elements or method steps may be implemented by e.g. a single unit or processor. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A dry-powder inhaler (10) comprising a casing (19), the casing (19) comprising:
a first casing portion (11);
a second casing portion (12);
a hinge arrangement (40) connecting said first casing portion (11) and second casing portion (12);
whereby the dry-powder inhaler (10) is assembled by means of folding the first casing portion (11) and the second casing portion (12) together;
whereby the first casing portion (11) comprises a cavity (81) for receiving the dose blister foil (95), said cavity (81) being adapted to contain a dry-powder medicament; and
whereby the first casing portion (11) and second casing portion (12) when folded together are adapted to form an inlet (9) for allowing a lidding foil (95) extending out there from, and
whereby the casing (19) further comprises an outlet nozzle (8) for providing the medicament, said outlet nozzle (8) extending at an angle (α) from one of said first and second casing portions (11, 12), further comprising a flow directing shoulder (35) adapted to direct the medicament into the nozzle outlet (8), said flow directing shoulder (35) being disposed inside the casing (19) between the cavity (81) and hinge arrangement (40), **characterized in that** the flow directing shoulder (35) is horse shoe shaped and adapted to enclose the nozzle outlet (8) when the first and second casing portions (11, 12) are folded together.

2. The inhaler (10) according to claim 1, whereby the outlet nozzle (8) is a tubular outlet nozzle (8), preferably being tapered with a wider end adjacent to the casing (19).

3. The inhaler (10) according to claim 1 or 2, whereby the dry-powder inhaler (10) comprises a lidding foil (95) being removably attached to the first casing portion (11) so as to seal the cavity (81).

4. The inhaler (10) according to any one of claims 1 to 3, whereby the first casing portion (12) comprises a protruding shoulder (45), said protruding shoulder (45) being adapted to receive the lidding foil (95).

5. The inhaler (10) according to any one of the preceding claims, whereby the nozzle outlet (8) is arranged in the second casing portion (12) of the casing (19).

6. The inhaler (10) according to any one of the preceding claims, whereby the angle (α) is at least 45 degrees, preferably said angle (α) being from 70 to 90 degrees, such as 70, 80, or 90 degrees.

7. The inhaler (10) according to any one of the preceding claims, further comprising flow directing elements (30) adapted to disaggregate the medicament, said flow directing elements (30) being disposed inside the casing (19) between the cavity (81) and the nozzle outlet (8).

8. The inhaler (10) according to claim 7, whereby the flow directing elements (30) protrudes from the first casing portion (11).

9. The inhaler (10) according to any one of the preceding claims, whereby the flow directing shoulder (35) further comprises a sloped internal surface (36), said sloped internal surface (36) facing the nozzle outlet (8) when the first and second casing portions (11, 12) are folded together.

10. The inhaler (10) according to any one of the preceding claims, whereby the second casing portion (12) or the first casing portion (11) comprises a set of inner walls (14) protruding from said second casing portion (12), the set of inner walls (14) extending parallel along the second casing portion (12), whereby each of the inner walls of the set of inner walls (14) has at least one inwardly directing portion (15) adapted to direct a flow inwardly towards the nozzle outlet (8).

11. The inhaler (10) according to any one of the preceding claims, whereby the second casing portion (12) comprises a flow directing member (48) adapted to direct flow entering through the inlet (9) towards the cavity (81).

12. The inhaler (10) according to any one of the preceding claims, whereby one of the casing portions (11, 12) comprises at least one latching knob (22) and the other casing portion (11, 12) comprises at least one aperture (21), the at least one aperture (21) being adapted to receive the at least one latching knob (22) so as to retain the casing portions (11, 12) in a folded position.

13. The inhaler (10) according to any one of the preceding claims, whereby the hinge arrangement (40) comprises a folding portion (42) comprising support members (41) protruding therefrom, whereby the support members (42) are adapted to abut to the first casing portion (11) and the second casing portion (12) when said casing portions (11, 12) are folded together.

## Patentansprüche

1. Trockenpulverinhalator (10), der ein Gehäuse (19) aufweist, wobei das Gehäuse (19) Folgendes aufweist:
ein erstes Gehäuseteil (11),
ein zweites Gehäuseteil (12),
eine Scharnieranordnung (40), die das erste Gehäuseteil (11) mit dem zweiten Gehäuseteil (12) verbindet,
wobei der Trockenpulverinhalator (10) durch das Zusammenfalten des ersten Gehäuseteils (11) und zweiten Gehäuseteils (12) zusammengesetzt wird,
wobei das erste Gehäuseteil (11) einen Hohlraum (81) zur Aufnahme der Dosis-Blisterfolie (95) aufweist, wobei der Hohlraum (81) ausgebildet ist, ein Trockenpulvermedikament zu beinhalten, und
wobei das erste Gehäuseteil (11) und das zweite Gehäuseteil (12), wenn diese zusammengefaltet sind, dazu angepasst sind, einen Einlass (9) zu bilden, um das Herausragen einer Deckfolie (95) von dort heraus zu ermöglichen, und
wobei das Gehäuse (19) ferner eine Auslassdüse (8) aufweist, um das Medikament abzugeben, wobei die Auslassdüse (8) sich unter einem Winkel (α) von einem des ersten und zweiten Gehäuseteils (11, 12) erstreckt,
und ferner eine strömungsleitende Schulter (35) aufweist, die ausgebildet ist, um das Medikament in die Auslassdüse (8) zu leiten, wobei die strömungsleitende Schulter (35) im Gehäuse (19) zwischen dem Hohlraum (81) und der Scharnieranordnung (40) angeordnet ist, **dadurch gekennzeichnet, dass** die strömungsleitende Schulter (35) hufeisenförmig geformt ist und angepasst ist, die Auslassdüse (8) zu umschließen, wenn das erste und zweite Gehäuseteil (11, 12) zusammengefaltet sind.

2. Inhalator (11) gemäß Anspruch 1, wobei die Auslassdüse (8) eine rohrförmige Auslassdüse (8) ist, die vorzugsweise konisch zulaufend mit einem breiteren Ende benachbart zum Gehäuse (19) ausgebildet ist.

3. Inhalator (10) gemäß Anspruch 1 oder 2, wobei der Trockenpulverinhalator (10) eine Deckfolie (95) aufweist, die an dem Gehäuseteil (11) entfernbar befestigt ist, um die Hohlkammer (81) zu verschließen.

4. Inhalator (10) gemäß einem der vorhergehenden Ansprüche 1 bis 3, wobei das erste Gehäuseteil (12) eine hervorstehende Schulter (45) aufweist, wobei die hervorstehende Schulter (45) dazu angepasst ist, die Deckfolie (95) aufzunehmen.

5. Inhalator (10) gemäß einem der vorhergehenden Ansprüche, wobei die Auslassdüse (8) in dem zweiten Gehäuseteil (12) des Gehäuses (19) angeordnet ist.

6. Inhalator (10) gemäß einem der vorhergehenden Ansprüche, wobei der Winkel (α) mindestens 45°, vorzugsweise von 70° bis 90° beträgt, beispielsweise 70°, 80° oder 90°.

7. Inhalator (10) gemäß einem der vorhergehenden Ansprüche, wobei dieser ferner strömungsleitende Elemente (30) aufweist, die dazu angepasst sind, das Medikament zu disaggregieren, und wobei die strömungsleitenden Elemente (30) in dem Gehäuse (19) zwischen dem Hohlraum (81) und der Auslassdüse (8) angeordnet sind.

8. Inhalator (10) gemäß Anspruch 7, wobei die strömungsleitenden Elemente (30) von dem ersten Gehäuseteil (11) hervorstehen.

9. Inhalator (10) gemäß einem der vorhergehenden Ansprüche, wobei die strömungsleitende Schulter (35) eine geneigte Innenfläche (36) aufweist, wobei die geneigte Innenfläche (36) der Auslassdüse (8) zugewandt ist, wenn das erste und zweite Gehäuseteil (11, 12) zusammengefaltet sind.

10. Inhalator (10) gemäß einem der vorhergehenden Ansprüche, wobei der zweite Gehäuseteil (12) oder der erste Gehäuseteil (11) einen Satz Innenwände (14) aufweist, die von dem zweiten Gehäuseteil (12) vorstehen, wobei sich der Satz Innenwände (14) parallel entlang des zweiten Gehäuseteils (12) erstreckt, wobei jeder der Innenwände des Satzes Innenwände (14) mindestens einen nach innen gerichteten Abschnitt (15) aufweist, der dazu ausgebildet ist, die Strömung innenliegend zur Auslassdüse (8) zu leiten.

11. Inhalator (10) gemäß einem der vorhergehenden Ansprüche, wobei das zweite Gehäuseteil (12) ein strömungsleitendes Element (48) aufweist, das dazu angepasst ist, die durch den Einlass (9) eintretende Strömung zum Hohlraum (81) zu leiten.

12. Inhalator (10) gemäß einem der vorhergehenden Ansprüche, wobei eines der Gehäuseteile (11, 12) mindestens einen Rastknopf (22) aufweist und das andere Gehäuseteil (11, 12) mindestens eine Öffnung (21) aufweist, wobei die mindestens eine Öffnung (21) angepasst ist, um den mindestens einen Rastknopf (22) aufzunehmen, sodass die Gehäuseteile (11, 12) in der zusammengefalteten Position gehalten werden.

13. Inhalator (10) gemäß einem der vorhergehenden Ansprüche, wobei die Scharnieranordnung (40) einen Faltabschnitt (42) aufweist, der von diesem vorstehende Stützelemente (41) aufweist, wobei die Stützelemente (41) angepasst sind, an dem ersten Gehäuseteil (11) und dem zweiten Gehäuseteil (12) anzuliegen, wenn die Gehäuseteile (11, 12) zusammengefaltet sind.

## Revendications

1. Inhalateur à poudre sèche (10) comprenant un boîtier (19), le boîtier (19) comprenant :
une première partie de boîtier (11) ;
une seconde partie de boîtier (12) ;
un agencement de charnière (40) reliant ladite première partie de boîtier (11) et ladite seconde partie de boîtier (12) ;
dans lequel l'inhalateur à poudre sèche (10) est assemblé au moyen du pliage de la première partie de boîtier (11) et de la seconde partie de boîtier (12) ensemble ;
dans lequel la première partie de boîtier (11) comprend une cavité (81) pour recevoir la plaquette thermoformée de dose (95), ladite cavité (81) étant adaptée pour contenir un médicament en poudre sèche ; et
dans lequel la première partie de boîtier (11) et la seconde partie de boîtier (12), lorsqu'elles sont pliées ensemble, sont adaptées pour former une entrée (9) pour permettre à un opercule (95) de s'étendre à partir de là,
et
dans lequel le boîtier (19) comprend en outre une buse de sortie (8) pour fournir le médicament, ladite buse de sortie (8) s'étendant selon un angle (α) à partir de l'une desdites première et seconde parties de boîtier (11, 12),
comprenant en outre un épaulement (35) de direction d'écoulement adapté pour diriger le médicament dans la sortie de buse (8), ledit épaulement (35) de direction d'écoulement étant disposé à l'intérieur du boîtier (19) entre la cavité (81) et l'agencement de charnière (40), **caractérisé en ce que** l'épaulement (35) de direction d'écoulement est en forme de fer à cheval et adapté pour enfermer la sortie de buse (8) lorsque les première et seconde parties de boîtier (11, 12) sont pliées ensemble.

2. Inhalateur (10) selon la revendication 1, dans lequel la buse de sortie (8) est une buse de sortie (8) tubulaire, de préférence effilée avec une extrémité plus large adjacente au boîtier (19).

3. Inhalateur (10) selon la revendication 1 ou 2, dans lequel l'inhalateur à poudre sèche (10) comprend un opercule (95) étant fixé de manière amovible à la première partie de boîtier (11) de manière à sceller la cavité (81).

4. Inhalateur (10) selon l'une quelconque des revendications 1 à 3, dans lequel la première partie de boîtier (12) comprend un épaulement saillant (45), ledit épaulement saillant (45) étant adapté pour recevoir l'opercule (95).

5. Inhalateur (10) selon l'une quelconque des revendications précédentes, dans lequel la sortie de buse (8) est agencée dans la seconde partie de boîtier (12) du boîtier (19).

6. Inhalateur (10) selon l'une quelconque des revendications précédentes, dans lequel l'angle (α) est d'au moins 45 degrés, de préférence ledit angle (α) étant de 70 à 90 degrés, tel que 70, 80 ou 90 degrés.

7. Inhalateur (10) selon l'une quelconque des revendications précédentes, comprenant en outre des éléments (30) de direction d'écoulement adaptés pour désagréger le médicament, lesdits éléments (30) de direction d'écoulement étant disposés à l'intérieur du boîtier (19) entre la cavité (81) et la sortie de buse (8).

8. Inhalateur (10) selon la revendication 7, dans lequel les éléments (30) de direction d'écoulement font saillie à partir de la première partie de boîtier (11).

9. Inhalateur (10) selon l'une quelconque des revendications précédentes, dans lequel l'épaulement (35) de direction d'écoulement comprend en outre une surface interne (36) inclinée, ladite surface interne (36) inclinée faisant face à la sortie de buse (8) lorsque les première et seconde parties de boîtier (11, 12) sont pliées ensemble.

10. Inhalateur (10) selon l'une quelconque des revendications précédentes, dans lequel la seconde partie de boîtier (12) ou la première partie de boîtier (11) comprend un ensemble de parois intérieures (14) faisant saillie à partir de ladite seconde partie de boîtier (12), l'ensemble de parois intérieures (14) s'étendant parallèlement le long de la seconde partie de boîtier (12), dans lequel chacune des parois intérieures de l'ensemble de parois intérieures (14) présente au moins une partie de direction vers l'intérieur (15) adaptée pour diriger un écoulement vers l'intérieur vers la sortie de buse (8).

11. Inhalateur (10) selon l'une quelconque des revendications précédentes, dans lequel la seconde partie de boîtier (12) comprend un organe (48) de direction d'écoulement adapté pour diriger l'écoulement entrant à travers l'entrée (9) vers la cavité (81).

12. Inhalateur (10) selon l'une quelconque des revendications précédentes, dans lequel l'une des parties de boîtier (11, 12) comprend au moins un bouton de verrouillage (22) et l'autre partie de boîtier (11, 12) comprend au moins une ouverture (21), l'au moins une ouverture (21) étant adaptée pour recevoir l'au moins un bouton de verrouillage (22) de manière à retenir les parties de boîtier (11, 12) dans une position pliée.

13. Inhalateur (10) selon l'une quelconque des revendications précédentes, dans lequel l'agencement de charnière (40) comprend une partie de pliage (42) comprenant des organes de support (41) saillant de celle-ci, dans lequel les organes de support (42) sont adaptés pour venir en butée contre la première partie de boîtier (11) et la seconde partie de boîtier (12) lorsque lesdites parties de boîtier (11, 12) sont pliées ensemble.
